# FASCICULE DE BREVET EUROPEEN

(11) **EP 1 383 762 B1**
(45) Date de publication et mention de la délivrance du brevet: **20.06.2007**
(21) Numéro de dépôt: 02727676.5
(22) Date de dépôt: 19.04.2002
(51) Int. Cl.: C07D 401/06

(54) **TETRAHYDROPYRIDYL-ALKYL-HETEROCYCLES, PROCEDE POUR LEUR PREPARATION ET COMPOSITIONS PHARMACEUTIQUES LES CONTENANTS**
TETRAHYDROPYRIDYL-ALKYL-HETEROZYKLE, VERFAHREN ZU IHRER HERSTELLUNG UND PHARMAZEUTISCHE ZUSAMMENSETZUNGEN DIE SIE ENTHALTEN
TETRAHYDROPYRIDYL-ALKYL-HETEROCYCLES, METHOD FOR PREPARING SAME AND PHARMACEUTICAL COMPOSITIONS CONTAINING SAME

(30) Priorité: 20.04.2001 FR 0105360; 20.04.2001 FR 0105362
(43) Date de publication de la demande: 28.01.2004
(73) Titulaire: Sanofi-Aventis, 75013 Paris (FR)
(72) Inventeur: BARONI, Marco, I-20010 Vanzago (IT); BOURRIE, Bernard, F-34980 Saint Gély du Fesc (FR); CASELLAS, Pierre, F-34090 Montpellier (FR)
(74) Mandataire: Varady, Peter
(86) Numéro de dépôt international: PCT/FR2002/001343
(87) Numéro de publication internationale: WO 2002/085888

(56) Documents cités:
- EP-A- 0 164 633
- WO-A-97/01536
- WO-A-98/48802
- DATABASE CA [en ligne] CHEMICAL ABSTRACTS SERVICE, COLUMBUS, OHIO, US; BOURRIE, BERNARD ET AL: "The neuroprotective agent SR 57746A abrogates experimental autoimmune encephalomyelitis and impairs associated blood-brain barrier disruption: implications for multiple sclerosis treatment" retrieved from STN Database accession no. 132:30802 XP002142809 & PROC. NATL. ACAD. SCI. U. S. A. (1999), 96(22), 12855-12859,

## Description

La présente invention concerne de nouveaux tétrahydropyridyl-alkyl-hétérocycles, les compositions pharmaceutiques les contenant et un procédé pour leur préparation.

US 5,118,691 et US 5,620,988 décrivent des tétrahydro-pyridines, substituées par un radical quinolyl-3-alkyle, montrant une activité dopaminergique.

Dukic, S. et al. (Arch. Pharm., 1997, 330(1/2): 25-28) décrivent des phényl-pipéridines portant un substituant benzimidazol-5-yl-éthyle, montrant également une activité dopaminergique.

EP-164633 décrit des tétrahydro-pyridines, substituées par un radical 4-indolyle, montrant une activité au niveau du système nerveux central.

WO 98/48802 décrit des tétrahydropyridyl-alkyl-phényle ou naphtyle utiles dans le traitement des maladies entraînant une démyélinisation.

Chem. Abs. 132: 30802 divulgue que le composé de l'exemple 37 du document précédent a également une activité modulatrice sur le TNF-alpha.

Il a été maintenant trouvé que certaines phényl- et pyridyl-tétrahydropyridines, substituées par des hétérocycles azotés, possèdent une puissante activité vis-à-vis de la modulation du TNF-alpha (de l'anglais Tumour Necrosis Factor).

Le TNF-alpha est une cytokine qui a récemment suscité de l'intérêt en tant que médiateur de l'immunité, de l'inflammation, de la prolifération cellulaire, de la fibrose etc. Ce médiateur est copieusement présent dans le tissu synovial enflammé et exerce un rôle important dans la pathogenèse de l'autoimmunité (Annu. Rep. Med. Chem., 1997, 32:241-250).

Ainsi, la présente invention concerne, selon un de ses aspects, des tétrahydropyridyl-alkyl-hétérocycles de formule (I) : 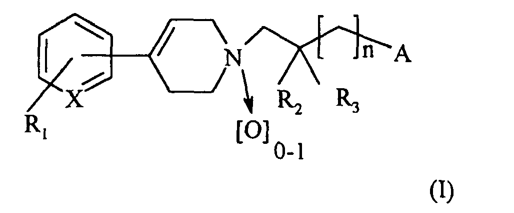 dans laquelle
- X: représente N ou CH ;
- R₁: représente un atome d'hydrogène ou d'halogène ou un groupe CF₃ ;
- R₂ et R₃: représentent indépendamment un atome d'hydrogène ou un groupe méthyle ;
- n: est 0 ou 1 ;
- A: représente un hétérocycle azoté de formule (a)-(1): 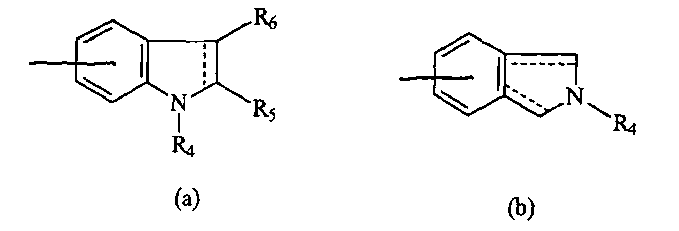 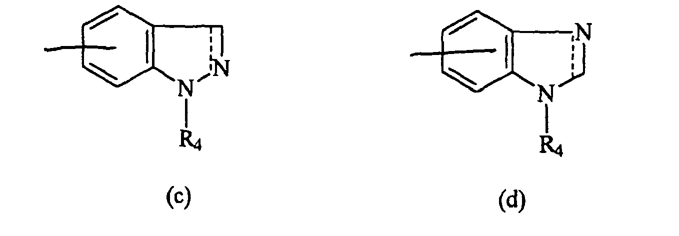 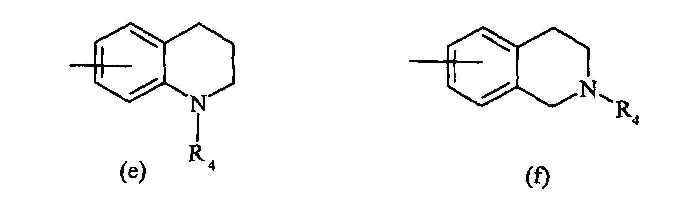 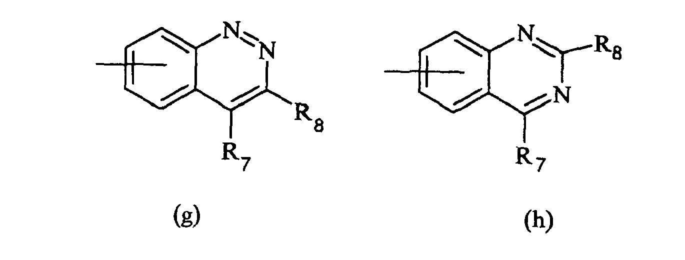 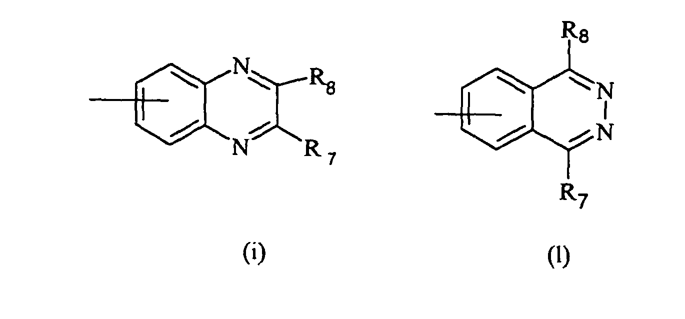
où R₄, R₅ et R₆ représentent chacun indépendamment un atome d'hydrogène ou un groupe (C₁-C₄) alkyle ;
les lignes pointillées représentent des liaisons simples ou doubles ;
R₇ et R₈ représentent chacun indépendamment un atome d'hydrogène ou d'halogène, un groupe (C₁-C₄) alkyle ou (C₁-C₄) alcoxy ;
ainsi que leurs N-oxydes et leurs sels ou solvates,
à la condition que lorsque X est CH, alors (a) n'est pas un résidu indol-4-yle.

Dans la présente description, le terme "(C₁-C₄)alkyle" désigne un radical monovalent d'un hydrocarbure en C₁-C₄ saturé à chaîne droite ou ramifiée et le terme "(C₁-C₄)alcoxy" désigne un radical monovalent d'un hydrocarbure en C₁-C₄ saturé à chaîne droite ou ramifiée lié par l'intermédiaire d'un atome d'oxygène.

Dans la présente description, le terme "halogène" désigne un atome choisi parmi le chlore, le brome, le iode et le fluor.

Lorsque les lignes pointillées sont des liaison doubles, les hétérocycles (a) à (d) représentent des hétérocycles aromatiques.

Des composés de formule (I) préférés sont ceux où n est zéro.

D'autres composés préférés sont ceux où R₂ et R₃ sont chacun un atome d'hydrogène.

D'autres composés préférés sont ceux où R₁ est un groupe CF₃.

D'autres composés préférés sont ceux où X est CH et R₁ est dans la position 2 ou 3 du benzène.

D'autres composés préférés sont ceux où X est CH et R₁ est un groupe CF₃.

D'autres composés préférés sont ceux où X est N et la pyridine est substituée dans les positions 2,6.

D'autres composés préférés sont ceux où R₄, R₅ et R₆ représentent chacun un hydrogène ou un groupe méthyle.

D'autres composés préférés sont ceux où R₇ et R₈ représentent chacun un atome d'hydrogène ou un groupe (C₁-C₄)alkyle.

D'autres composés préférés sont ceux où A est un radical de formule (a)-(d), les composés où A est un radical de formule (a) étant particulièrement préférés.

D'autres composés préférés sont ceux où A est un radical de formule (a)-(d), notamment le radical (a), et les lignes pointillées représentent des liaisons doubles.

Les noyaux de formule (a)-(d) où les lignes pointillées représentent des liaisons doubles, pourront dans la présente description être dénommés respectivement, indole, isoindole, indazole et benzimidazole. Les noyaux de formule (a)-(d) où les lignes pointillées représentent des liaisons simples, pourront dans la présente description être dénommés respectivement, indoline, isoindoline, indazoline et benzimidazoline. Ces noyaux peuvent être rattachées au reste de la molécule de formule (I) par l'un quelconque des atomes de carbone des positions 4, 5, 6 ou 7.

Les noyaux de formule (e)-(f) pourront dans la présente description être dénommés respectivement, tétrahydro-quinoléine et tétrahydro-isoquinoléine. Ces noyaux peuvent être rattachées au reste de la molécule de formule (I) par l'un quelconque des atomes de carbone des positions 5, 6, 7 ou 8.

D'autres composés préférés sont ceux où A est une 1,4-benzodiazine de formule (i).

Les noyaux de formule (g)-(1) pourront dans la présente description être alternativement dénommés soit 1,2- , 1,3-, 1,4- et 2,3-benzodiazines soit, respectivement, cinnoline, quinazoline, quinoxaline et phtalazine. Ces benzodiazines peuvent être rattachées au reste de la molécule de formule (I) par l'un quelconque des atomes de carbone des positions 5, 6, 7 ou 8.

Selon la présente invention, les composés de formule (I) peuvent exister comme dérivés N-oxydes. Comme indiqué dans la formule ci-dessus, les composés de formule (I) peuvent notamment porter le groupe N-oxyde sur la tétrahydropyridine ou bien sur un ou les deux azotes des groupes (a)-(1), ou bien tous les azotes présents peuvent être simultanément oxydés.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

Les stéréoisomères optiquement purs, ainsi que les mélanges d'isomères des composés de formule (I), dus au carbone asymétrique, quand l'un de R₂ et R₃ est un méthyle et l'autre un hydrogène, dans une proportion quelconque, font partie de la présente invention.

Les composés de formule (I) peuvent être préparés par une réaction de condensation à partir d'un composé de formule (II) : 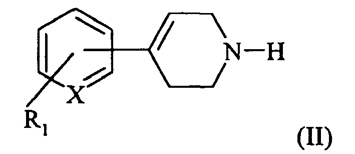 dans laquelle X et R₁ sont tels que définis ci-dessus, avec un composé de formule (III): 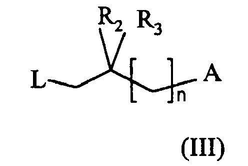 dans laquelle R₂, R₃, n et A sont tels que définis précédemment et L est un groupe partant, isolement du composé de formule (I) et transformation éventuelle en un de ses sels ou solvates ou dans ses dérivés N-oxydes.

La réaction de condensation est normalement conduite en mélangeant les composés de départ (II) et (III) dans un solvant organique inerte, selon les méthodes conventionnelles.

Par "solvant organique inerte" on entend un solvant qui n'interfère pas avec la réaction. De tels solvant sont par exemple les alcools tel que le méthanol, l'éthanol, l'isopropanol ou le butanol, ou les cétones, tels que l'isobutyl-metil-cétone, ce dernier étant un solvant particulièrement préféré.

Comme groupe partant L on peut par exemple utiliser un atome de chlore ou de brome ou bien un groupe mésyloxy (CH₃-SO₂-O-).

La réaction est conduite à une température comprise entre -10°C et la température de reflux du mélange réactionnel, la température de reflux étant préférée.

La réaction peut être convenablement conduite en présence d'un accepteur de protons, par exemple d'un carbonate alcalin ou d'une amine tertiaire telle que la triéthylamine.

La réaction est normalement terminée après quelques heures, normalement de 1 à 12 heures suffisent pour compléter la condensation.

Le composé voulu est isolé selon les techniques conventionnelles sous forme de base libre ou d'un de ses sels. La base libre peut être transformée dans un de ses sels par simple salification dans un solvant organique tel qu'un alcool, de préférence l'éthanol ou l'isopropanol, un éther comme le 1,2-diméthoxyéthane, l'acétate d'éthyle, l'acétone ou un hydrocarbure comme l'hexane.

Le composé de formule (I) obtenu est isolé selon les techniques usuelles et éventuellement transformé en un de ses sels ou solvates ou dans ses dérivés N-oxyde.

Les composés de départ de formule (II) sont connus ou bien ils peuvent être préparés de façon analogue aux composés connus.

Les composés de départ de formule (III) sont connus ou bien ils peuvent être préparés à partir des acides ou esters correspondants, par réduction du groupe carboxylique en groupe alcool et substitution de l'OH par le groupe L souhaité selon les méthodes conventionnelles. Des exemples de telles réactions sont consignés dans la partie expérimentale.

Alternativement, es composés de départ de formule (III) peuvent être préparés par réaction des dérivés organo-métalliques des noyaux (a)-(1) de formule (IV)

M-A (IV)

où
M représente un radical métallique et A est tel que défini ci-dessus, avec un éther cyclique approprié, pour obtenir l'alcool de formule (V) 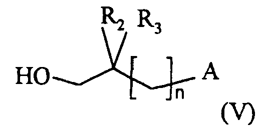 et substitution de l'OH par le groupe L souhaité selon les méthodes conventionnelles.

Par "radical métallique" on entend selon la présente invention, un radical contenant au moins un métal et qui est apte à ouvrir un éther cyclique approprié afin de donner l'alcool de formule (V). Des composés de formule (IV) appropriés sont par exemple les composés de Grignard de formule Hal-Mg-A et les cuprates Hal-Cu-A, où Hal est tel que défini précédemment, ainsi que les dérivés du lithium Li-A, etc.

Les composés organo-métalliques de formule (IV) sont préparés selon les techniques bien connues, par exemple à partir des dérivés halogénés des noyaux A ci-dessus par réaction avec du magnésium ou un dérivé du lithium ; lorsqu'un veut utiliser un lithium-dérivé du composé de formule (IV), il est convenable de former un cuprate dudit composé (IV), par exemple par réaction avec du CuI, avant d'effectuer la réaction d'ouverture de l'éther cyclique approprié. Des exemples de telles réactions, qui sont bien connues à l'homme du métier, sont consignés dans la partie expérimentale.

Les éthers cycliques sont choisis en fonction du composé de formule (I) qu'on souhaite obtenir. Lorsque par exemple on veut préparer un composé de formule (I) où R₂ et R₃ sont l'hydrogène et n est zéro, l'éther cyclique approprié est l'oxyde d'éthylène.

Alternativement, selon les significations de R₂, R₃ et n, des cétones ou des aldéhydes peuvent être utilisés au lieu des éthers cycliques.

Les composés de formule (I) peuvent également être préparés par un procédé qui prévoit :
(a) de faire réagir le composé de formule (VI): 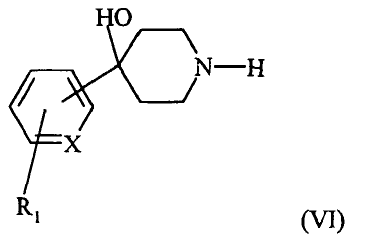 dans laquelle X et R₁ sont définis comme précédemment, avec un dérivé fonctionnel de l'acide de formule (VII) 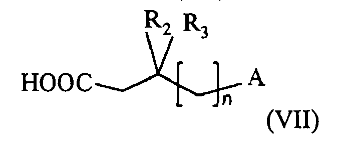 dans laquelle R₂, R₃, n et A sont tels que définis ci-dessus,
(b) de réduire le groupe carbonyle du composé de formule (VIII) ainsi obtenu: 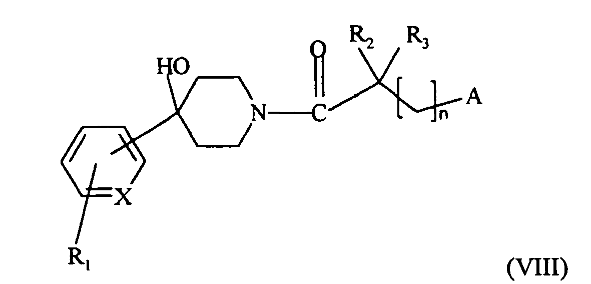
(c) de déshydrater le pipéridinol intermédiaire de formule (IX) ainsi obtenu: 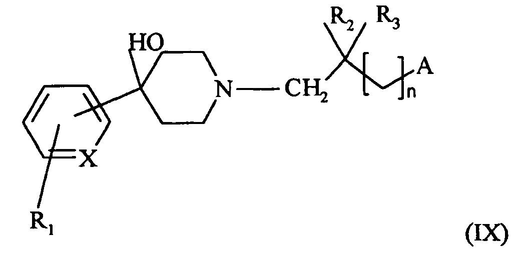
(d) d'isoler le composé de formule (I) ainsi obtenu et, éventuellement le transformer en l'un de ses sels ou solvates ou dans ses dérivés N-oxyde.

La réaction de l'étape (a) peut être convenablement conduite dans un solvant organique à une température comprise entre -10°C et la température de reflux du mélange réactionnel.

Il peut être préférable de réaliser la réaction à froid lorsqu'elle est exothermique, comme dans le cas où on utilise le chlorure en tant que dérivé fonctionnel de l'acide de formule (VII).

Comme dérivé fonctionnel approprié de l'acide de formule (VII), on peut utiliser l'acide libre, éventuellement activé (par exemple avec le BOP = tri(diméthylamino)benzotriazol-1-yloxyphosphonium hexafluorophosphate), un anhydride, un anhydride mixte, un ester actif ou un halogénure d'acide, le bromure de préférence. Parmi les esters actifs, l'ester de p-nitrophényle est particulièrement préféré, mais les esters de méthoxyphényle, de trityle, de benzhydryle et similaires sont également convenables.

Comme solvant de réaction, on utilise de préférence un solvant halogéné tel que le chlorure de méthylène, le dichloroéthane, le 1,1,1-trichloroéthane, le chloroforme et similaires, mais aussi d'autres solvants organiques compatibles avec les réactifs employés, par exemple le dioxane, le tétrahydrofurane ou un hydrocarbure tel que l'hexane, peuvent être également employés.

La réaction peut être convenablement conduite en présence d'un accepteur de protons, par exemple d'un carbonate alcalin ou d'une amine tertiaire telle que la triéthylamine.

La réduction de l'étape (b) peut être convenablement réalisée par des agents de réduction appropriés tels que les complexes du borane, par exemple le diméthylsulfure de borane ([CH₃]₂S-BH₃), les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofurane (THF), le dioxane ou le 1,2-diméthoxyéthane.
Dans les réactions décrites ci-dessus, les atomes d'azote présents sur les noyaux A sont préférablement protégés par des groupes protecteurs conventionnels. Comme groupes protecteurs, on peut bien utiliser les groupes protecteurs usuels pour les atomes d'azote tels que par exemple le *tert-*butoxycarbonyle, l'acétyle, le benzyloxycarbonyle ou le tri-isopropyl-silyle, ce dernier étant particulièrement préféré. Ces groupes protecteurs sont enfin clivés pour donner les composés de formule (I).

Les composés de formule (I) portant un groupe N-oxyde sur les atomes d'azote des groupes (a)-(1) peuvent être préparés à partir des dérivés N-oxyde des composés de formule (VII).

Les composés de formule (I) portant un groupe N-oxyde sur l'atome d'azote de la tétrahydropyridine peuvent être préparés par oxydation des composés de formule (I) correspondant. Dans ce cas, le composé de formule (I) tel qu'obtenu par exemple par la synthèse ci-dessus, est soumis à une réaction d'oxydation selon les méthodes conventionnelles, par exemple à une réaction avec de l'acide m-chloro-perbenzoïque dans un solvant convenable et isolé selon les techniques usuelles bien connues à l'homme du métier.

Les composés de l'invention possèdent des propriétés intéressantes vis-à-vis de l'inhibition du TNF-α.

Ces propriétés ont été mises en évidence à l'aide d'un test visant à mesurer l'effet de molécules sur la synthèse du TNF-α induite chez la souris Balb/c par du lipopolysaccharide (LPS) d'Escherichia Coli (055 :B5, Sigma, St Louis, Mo).

Les produits à tester sont administrés par voie orale à des groupes de 5 souris Balb/c femelles (Charles River, France) âgées de 7 à 8 semaines. Une heure après, le LPS est administré par voie intraveineuse (10µg/souris). Le sang de chaque animal est prélevé 1,5 heure après l'administration du LPS. Les échantillons sont centrifugés, le plasma est récupéré et congelé à -80°C. Le TNF-α est mesuré à l'aide de kits commerciaux (R et D, Abingdon, UK).

Dans ce test, des composés représentatifs de l'invention se sont montrés très actifs, en inhibant la synthèse du TNF-α même à doses très faibles.
Grâce à cette activité et à leur faible toxicité, les composés de formule (I), y compris les composés de formule (I) où X est CH, et (a) est un résidu indol-4-yle, et leurs sels ou solvates, peuvent bien être utilisés dans le traitement des maladies liées à des troubles immunitaires et inflammatoires. Notamment les composés de formule (I) peuvent être utilisés pour traiter l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations.

Les composés de formule (I) et leurs sels et solvates pharmaceutiquement acceptables sont de préférence administrés par voie orale.

Dans les compositions pharmaceutiques de la présente invention par voie orale, le principe actif peut être administré sous formes unitaires d'administration, en mélange avec des supports pharmaceutiques classiques, aux animaux et aux êtres humains pour le traitement des affections susmentionnées. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend comme toujours du degré d'avancement de la maladie ainsi que de l'âge et du poids du patient. Néanmoins, les doses unitaires comprennent généralement de 0,001 à 100 mg, mieux de 0,01 à 50 mg, de préférence de 0,1 à 20 mg de principe actif, avantageusement de 0,5 à 10 mg.

Selon un autre de ses aspects, la présente invention concerne une association comprenant un composé de formule (I) ou l'un de ses sels ou solvates pharmaceutiquement acceptables, et au moins un composé choisi parmi les agents immunosuppresseurs, tel que l'interféron bêta-1b; l'hormone adrénocorticotrope; les glucocorticoïdes tels que la prednisone ou la méthylprednisolone; les inhibiteurs de l'interleukine-1.

Plus particulièrement, l'invention concerne une association comprenant un composé de formule (I), ou l'un de ses sels ou solvates pharmaceutiquement acceptables et au moins un composé choisi parmi le roquinimex (1,2-dihydro-4-hydroxy-N,1-diméthyl-2-oxo-3-quinolinecarboxanilide), le myloran (produit de la société Autoimmune contenant de la myéline bovine), l'antegren (anticorps humain monoclonal des sociétés Elan/Athena Neurosciences) l'interféron bêta-1b recombinant.

D'autres associations possibles sont celles constituées par un composé de formule (I) ou l'un de ses sels ou solvates pharmaceutiquement acceptables et un bloqueur des canaux potassiques, tel que par exemple la fampridine (4-aminopyridine).

Selon un autre de ses aspects, l'invention concerne l'utilisation des composés de formule (I) pour la préparation de médicaments pour le traitement des maladies liées à des troubles immunitaires et inflammatoires, notamment l'athérosclérose, les maladies autoimmunes, les maladies qui entraînent la démyélinisation des neurones (telles que la sclérose en plaques), l'asthme, l'arthrite rhumatoïde, les maladies fibrotiques, la fibrose idiopathique pulmonaire, la fibrose cystique, la glumérulonéphrite, la spondylite rhumatoïde, l'ostéoarthrite, la goutte, la résorption osseuse et cartilagineuse, l'ostéoporose, la maladie de Paget, le myélome multiple, l'uvéorétinite, les chocs septiques, la septicémie, les chocs endotoxiniques, la réaction du greffon contre l'hôte, le rejet des greffes, le syndrome de détresse respiratoire de l'adulte, la silicose, l'asbestose, la sarcoïdose pulmonaire, la maladie de Crohn, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospasme coronarien, angine de poitrine, insuffisance cardiaque, l'attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, le diabète, la cachexie, le cancer, les dommages liés aux radiations, comprenant l'administration d'un composé de formule (I), y compris les composés de formule (I) où X est CH, et (a) est un résidu indol-4-yle, ou de l'un de ses sels ou solvates pharmaceutiquement acceptables, seul ou en association avec d'autres principes actifs.

Les exemples qui suivent illustrent l'invention.

### PREPARATION 1

### 2-(1-Triisopropyl-silyl)-1H-indol-5-yl)éthyl-méthansulfonate

On dissout, en atmosphère d'azote et à la température de -78°C, 1 g de 5-bromo-indole (10,2 mmole) dans 70 ml de THF anhydre et on y ajoute, goutte à goutte, 15,8 ml (15,81 mmole) de LiN(SiCH₃)₂ sous forme d'une solution 1M dans du THF et après toujours à -78°C et avec précaution, 3,9 ml de chlorure de triisopropyl-silyle (TIPSiCI) (18,36 mmole). On agite à -78°C pendant 15 minutes puis à la température ambiante pendant une nuit. On verse dans un mélange constitué par 100 ml d'une solution tampon à pH 7 et 100 ml de « brine », on extrait à l'éther diéthylique, on lave la phase organique avec de l'acide chlorhydrique 1 M et avec de l'eau, on sèche sur du sulfate de sodium et on évapore le solvant sous pression réduite. On purifie le produit par chromatographie sur colonne de gel de silice en éluant avec de l'hexane. On dissout 3 g du produit ainsi obtenu dans 30 ml de THF à -78°C et on y ajoute 13,4 ml de BuLi en solution 1,6N dans de l'hexane (21,5 mmole). On agite à la température ambiante pendant 1 heure on refroidit encore à -78°C et on verse dans une suspension de 647 mg de CuI (3,4 mmole) dans un mélange de 20 g d'oxyde d'éthylène et 20 ml de THF, à -78°C. On agite à -40°C pendant 5 heures et on y ajoute, goutte à goutte, 43 ml d'une solution de tampon phosphate à pH 7 ; on dilue avec 17 ml d'eau froide et on filtre sur célite. On lave la solution filtrée avec 43 ml de NH₃ 5M et avec de l'eau froide, on sèche la phase organique sur de sulfate de sodium et on évapore le solvant sous pression réduite. On purifie le brut par chromatographie sue colonne de gel de silice en éluant par un mélange acétate d'éthyle/hexane = 3/7. On obtient ainsi 1,7 g de 5-(2-hydroxyéthyl)-1-(triisopropyl-silyl)-1H-indole. On dissout 1 g de l'alcool ainsi obtenu sous atmosphère d'azote dans 15 ml de chlorure de méthylène et on ajoute 0,44 ml de triéthylamine, 384 mg de 4-diméthylamino-pyridine (DMAP) et 0,243 ml de chlorure de mésyle. On agite à la température ambiante pendant 2 heures, on y ajoute 15 ml de H₃PO₄ à 3%. On lave la phase organique avec une solution aqueuse saturée en bicarbonate de sodium, on sèche la phase organique et on évapore le solvant sous pression réduite. On obtient ainsi 1,25 g du produit du titre.

### PREPARATION 2

### 2-(1-Triisopropyl-silyl)-1H-indol-6-yl)éthyl-méthansulfonate

En opérant comme décrit dans la Préparation 1 mais en utilisant le de 6-bromo-indole au lieu du de 5-bromo-indole, on obtient le composé du titre.

### PREPARATION 3

### Acide 1H-benzimidazol-6-yl-acétique

On dissout 1,9 g (9,04 mmole) de (4-amino-3-nitrophényl)acétate de méthyle (J. Med. Chem., 1997, 40(7) : 1049) dans 300 ml de méthanol et on y ajoute 480 mg de Pd/C à 10%. On hydrogène à la température ambiante pendant 6 heures, on filtre le catalyseur et on évapore le solvant sous pression réduite. On obtient ainsi 1,6 g de huile qu'on dissout dans 18 ml d'acide formique à 98% et on chauffe à 100°C pendant 5 heures. On évapore ensuite le solvant sous pression réduite, et on obtient ainsi 1,6 g du produit du titre.

Chromatographie sur couche mince (éluant acétate d'éthyle/méthanol =1/1): R.f. 0,35.

### PREPARATION 4

### 6-(2-Chloroéthyl)-quinoxaline

On dissout 190 mg (0,9 mmole) de (4-amino-3-nitrophényl)-acétate d'éthyle (obtenu comme décrit dans J. Med. Chem., 1997, 40(7)) dans 30 ml de méthanol et on y ajoute 48 mg de Pd/C à 10%. On hydrogène à la température ambiante pendant 6 heures. On filtre le catalyseur et on évapore le solvant en obtenant 160 mg d'huile. On dissout le produit ainsi obtenu dans 4 ml de méthanol, on y ajoute 160 ml de 1,4-dioxane-2,3-diol et on agite pendant deux heures à la température ambiante. On évapore le solvant et on purifie le résidu par flash-chromatographie sur colonne de gel de silice en éluant par un mélange cyclohexane/acétate d'éthyle=4/6 en obtenant ainsi l'ester éthylique de l'acide quinoxalin-6-yl acétique. On dissout le produit ainsi obtenu dans 3 ml d'éther diéthylique anhydre en courant d'azote. On y ajoute 55 mg (1,46 mmole) de LiAlH₄ en suspension dans 2 ml d'éther diéthylique. On agite à la température ambiante pendant 3,5 heure, on dilue à l'éther diéthylique, on verse dans un mélange eau/glace, on sépare les phases et on sèche la phase organique et on évapore le solvant. On obtient 110 mg de produit brut qu'on purifie par colonne de gel de silice en éluant avec de l'acétate d'éthyle. On obtient ainsi 31 mg de 6-(2-hydroxyéthyl)-quinoxaline qu'on dissout dans 0,5 ml de chlorure de méthylène anhydre en courant d'azote. On refroidit la solution à 0°C, on y ajoute goutte à goutte 0,3 ml de SOCl₂ et on agite pendant deux heures à 0°C et après une nuit à la température ambiante. On ajoute du chlorure de méthylène et de l'eau, on porte la solution à pH basique par addition de NaHCO₃, on extrait au chlorure de méthylène et on sépare les deux phases. On sèche la phase organique et on évapore le solvant. On obtient ainsi 34 mg du produit du titre.

### PREPARATION 5

### 6-(2-Chloroéthyl)-2,3-diméthylquinoxaline

En opérant comme décrit dans la Préparation 4 mais en utilisant la 1,3-butandione au lieu du 1,4-dioxane-2,3-diol on obtient le composé du titre.

### PREPARATION 6

### 6-(2-Bromoéthyl)-2,3-diéthylquinoxaline

En opérant comme décrit dans la Préparation 4 mais en utilisant la 3,4-hexandione au lieu du 1,4-dioxane-2,3-diol et en traitant l'alcool 6-(2-hydroxyéthyl)-2,3-diéthylquinoxaline intermédiaire ainsi obtenu par de l'acide bromhydrique au lieu du SOCl₂, on obtient le composé du titre.

### EXEMPLE 1

### 5-[2-(4-(3-trifluorométhyl-phényl)-1,2,3,6-tétrahydro-pyrid-1-yl)éthyl]-1H-indole

On agite pendant 10 minutes, à la température ambiante, un mélange de 1 g (3,78 mmole) de 4-hydroxy-4-(3-trifluorométhyl-phényl)-1,2,3,6-tétrahydropyridine et 1,2 ml de triéthylamine (8,71 mmole) dans 10 ml d'isopropanol. On y ajoute 1,2 g de 2-((1-tri-isopropyl-silyl)-1H-indol-5-yl)éthyl-méthansulfonate de la Préparation 1 dissout dans 10 ml d'isopropanol et on chauffe au reflux pendant 12 heures. On évapore ensuite le solvant sous pression réduite, on purifie le brut par chromatographie sur colonne de gel de silice en éluant par un mélange hexane/acétate d'éthyle= 7/3. On obtient ainsi le produit de condensation. On dissout 0,9 g du produit ainsi obtenu dans 15 ml de THF et on refroidit la solution à 0°C. On y ajoute 0,66 g de fluorure de tétrabutyl-ammonium (Bu₄NF · 3H₂O (2,1 mmole). On agite à la température ambiante pendant 1 heure, on évapore ensuite le solvant et on purifie le brut par chromatographie sur colonne de gel de silice en éluant par un mélange hexane/acétate d'éthyle= 4/6. On lave le produit obtenu dans de l'hexane, on filtre et on obtient ainsi 0,4 g du produit du titre.
P.f. 122-123°C.

### EXEMPLE 2

### 6-[2-(4-(3-trifluorométhyl-phényl)-1,2,3,6-tétrahydro-pyrid-1-yl)éthyl]-1H-indole

En opérant comme décrit dans l'Exemple 1 mais en utilisant le produit de la Préparation 2 au lieu du produit de la Préparation 1 on obtient le composé du titre.
P.f. 168-169°C.
Chromatographie sur couche mince (éluant cyclohexane/acétate d'éthyle=1/1): R.f. 0,2.

### EXEMPLE 3

### 6-[2-(4-(3-trifluorométhyl-phényl)-1,2,3,6-tétrahydro-pyrid-1-yl)éthyl]-1H-benzimidazole et son dichlorhydrate

### 3a) 1-[4-hydroxy-4-(3-trifluorométhyl-phényl)-1-pipéridinyl]-2-(6-benzimidazolyl)-1-éthanone

On agite pendant une nuit, à la température ambiante, un mélange de 1,46 g (0,0083 mole) d'acide 1H-benzimidazol-6-yl-acétique de la Préparation 3, 2,0 g (0,0081 mole) de 4-hydroxy-4-(3-trifluorométhyl-phényl)pipéridine, 3,6 g de B.O.P., 3,4 ml de triéthylamine dans 40 ml de diméthylformamide. On ajoute un mélange d'acétate d'éthyle et d'eau, on sépare les deux phases, on sèche la phase organique sur du sulfate de sodium et on évapore le solvant. On purifie le brut par chromatographie sur colonne de gel de silice en éluant par un mélange acétate d'éthyle/méthanol= 9/1 et on obtient ainsi le composé du titre.

### 3b) 6-[2-(4-hydroxy-4-(3-trifluométhyl-phényl)-pipéridin-1-yl)-éthyl]-1H-benzimidazole

On dissout dans 18 ml de THF anhydre et en courant d'azote, 1 g du produit obtenu par l'Exemple 3a, on chauffe au reflux pendant 30 minutes et y on ajoute 1 ml de diméthylsulfure de borane dans 13,5 ml de THF et on chauffe au reflux pendant 4 heures. On refroidit le mélange, on y ajoute 13,5 ml d'éthanol et on chauffe au reflux pendant 30 minutes. On évapore le solvant, on reprend le résidu dans le mélange acétate d'éthyle/NH₄OH dilué, on sépare les deux phases et on lave la phase organique à l'eau. On sèche sur du sulfate de sodium et on évapore le solvant sous pression réduite. On purifie le brut par chromatographie sur colonne de gel de silice en éluant par un mélange acétate d'éthyle/méthanol = 8/2. On obtient 0,540 g du produit du titre sous forme d'huile.

### 3c) 6-[2-(4-(3-trifluorométhyl-phényl)-1,2,3,6-tétrahydro-pyrid-1-yl)éthyl]-1H-benzimidazole et son dichlorhydrate

On dissout 0,540 g du produit de l'étape précédente dans 6,5 ml d'acide acétique, on y ajoute 1,5 ml d'acide sulfurique à 96% et on chauffe à 80°C pendant 1 heures. On verse dans de un mélange NaOH/glace et on extrait à l'acétate d'éthyle. On lave à l'eau, on sèche et on évapore sous pression réduite. On obtient ainsi le composé du titre. On prépare le sel dichlorhydrate à l'aide d'une solution d'isopropanol saturé en acide chlorhydrique.
P.f. (dichlorhydrate) 260-263°C.

### EXEMPLE 4

### 7-[2-(4-(3-trifluorométhyl-phényl)-1,2,3,6-tétrahydro-pyrid-1-yl)éthyl]-N-méthyl-1,2,3,4-tétrahydro-isoquinoléine et son dichlorhydrate hydraté

En opérant comme décrit dans l'Exemple 3 mais en utilisant l'acide N-méthyl-1,2,3,4-tétrahydro-isoquinol-7-yl-acétique au lieu de l'acide 1H-benzimidazol-6-yl-acétique on obtient le composé du titre.
P.f. (dichlorhydrate hydraté) 180°C.

### EXEMPLE 5

### 7-[2-(4-(3-tritluorométhyl-phényl)-1,2,3,6-tétrahydro-pyrid-1-yl)éthyl]-1,2,3,4-tétrahydro-isoquinoléine

On dissout 100 mg du produit de l'Exemple 4 (base, 30 mmole) dans 1,5 ml de chloroformiate de 1-chloroéthyle et on chauffe à 80°C pendant 6 heures. On ajoute 5 ml de méthanol et on chauffe le mélange au reflux pendant 1 heure. On évapore le solvant sous pression réduite et on purifie le produit brut par chromatografie sur colonne de gel de silice en éluant par du méthanol. On obtient ainsi le produit du titre.
Chromatographie sur couche mince: (éluant méthanol 100%): R.f. 0,4.

### EXEMPLE 6

### 6-[2-(4-(3-trifluorométhyl-phényl)-1,2,3,6-tétrahydro-pyrid-1-yl)éthyl]-4-méthoxy-quinazoline

En opérant comme décrit dans l'Exemple 3 mais en utilisant l'acide 4-méthoxy-quinazol-6-yl-acétique au lieu de l'acide 1H-benzimidazol-6-yl-acétique on obtient le composé du titre.

### EXEMPLE 7

### 6-[2-[4-(3-trifluoro-méthyl-phényl)-1,2,3,6-tétrahydro-pyrid-1-yl]-éthyl]-quinoxaline et son dichlorhydrate

On dissout 340 mg (1,78 mmole) de 6-(2-chloroéthyl)-quinoxaline de la Préparation 4 dans 12 ml d'isopropanol et on y ajoute 791 mg (3,56 mmole) de 4-(3-trifluoro-méthyl-phényl)-1,2,3,6-tétrahydropyridine. On chauffe au reflux pendant 4 heures et après on agite une nuit à la température ambiante. On évapore le solvant sous pression réduite et on obtient le produit brut qu'on purifie par chromatographie sur colonne de gel de silice en éluant par de l'acétate d'éthyle. On obtient ainsi le produit du titre. On prépare son sel dichlorhydrate par réaction avec de l'aide chlorhydrique dans de l'isopropanol.
P.f. (dichlorhydrate) 239-241°C.

### EXEMPLE 8

### 6-[2-[4-(3-trifluoro-méthyl-phényl)-1,2,3,6-tétrahydro-pyrid-1-yl]-éthyl]-2,3-diméthyl-quinoxaline et son dichlorhydrate

En opérant comme décrit dans l'Exemple 7 mais en utilisant la 6-(2-chloroéthyl)-2,3-diméthylquinoxaline de la Préparation 5, on obtient le composé du titre.
P.f. (dichlorhydrate) 209-212°C.

### EXEMPLE 9

### 6-[2-[4-(3-trifluoro-méthyl-phényl)-1,2,3,6-tétrahydro-pyrid-1-yl]-éthyl]-2,3-diéthyl-quinoxaline et son dichlorhydrate

En opérant comme décrit dans l'Exemple 7 mais en utilisant la 6-(2-bromoéthyl)-2,3-diéthylquinoxaline de la Préparation 6, on obtient le composé du titre.
P.f (dichlorhydrate) 210-212°C.

### EXEMPLE 10

### 6-[2-14-(6-trifluoro-méthyl-pyrid-2-yl)-1,2,3,6-tétrahydro-pyrid-1-yl]-éthyl]-quinoxaline et son oxalate

En opérant comme décrit dans l'Exemple 7 mais en utilisant la 4-(6-trifluoro-méthyl-pyrid-2-yl)-1,2,3,6-tétrahydropyridine au lieu de la 4-(3-trifluoro-méthyl-phényl)-1,2,3,6-tétrahydropyridine, on obtient le composé du titre.
P.f. (oxalate) 125-128°C.

### EXEMPLE 11

### 6-[2-[4-(3-trifluoro-méthyl-phényl)-1,2,3,6-tétrahydro-pyrid-1-yl]-éthyl]-phtalazine

En opérant comme décrit dans l'Exemple 3 mais en utilisant l'acide phtalaz-6-yl-acétique on obtient le composé du titre.
Chromatographie sur couche mince (éluant acétate d'éthyle/méthanol=8/2): R.f. 0,4).

## Revendications

1. Composé de formule (I) : 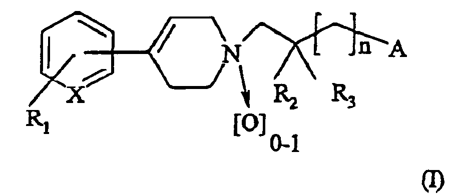 dans laquelle
X représente N ou CH ;
R₁ représente un atome d'hydrogène ou d'halogène ou un groupe CF₃ ;
R₂ et R₃ représentent indépendamment un atome d'hydrogène ou un groupe méthyle ;
n est 0 ou 1;
A représente un hétérocycle azoté de formule (a)-(1): 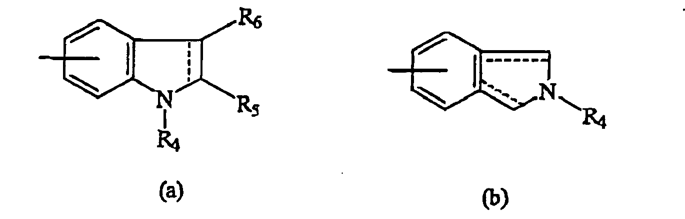 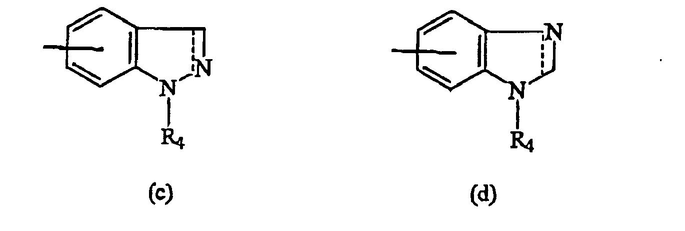 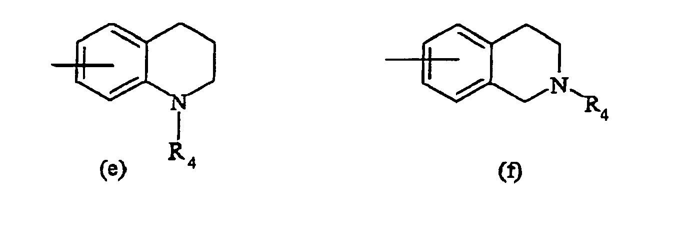 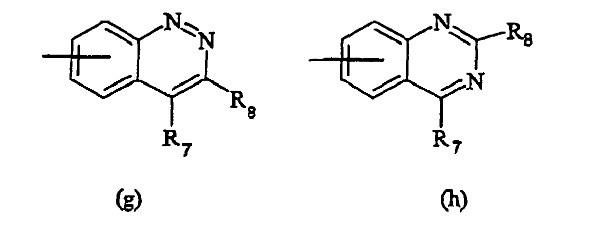 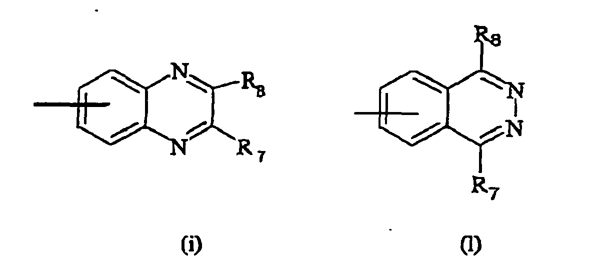
où R₄, R₅ et R₆ représentent chacun indépendamment un atome d'hydrogène ou un groupe (C₁-C₄) alkyle ;
les lignes pointillées représentent des liaisons simples ou doubles ;
R₇ et R₈ représentent chacun indépendamment un atome d'hydrogène ou d'halogène, un groupe (C₁-C₄) alkyle ou (C₁-C₄) alcoxy ;
ainsi que ses N-oxydes et ses sels ou solvates,
à la condition que lorsque X est CH, alors (a) n'est pas un résidu indol-4-yle.

2. Composé selon la revendication 1, où n est zéro.

3. Composé selon la revendication 1, où R₂ et R₃ sont chacun un hydrogène.

4. Composé selon le la revendication 1, où R₁ est un groupe CF₃.

5. Composé selon la revendication 1, où X est CH et R₁ est dans la position 3 du benzène.

6. Composé selon la revendication 1, où X est CH et R₁ est dans la position 2 du benzène.

7. Composé selon la revendication 1, où X est N et la pyridine est substituée dans les position 2,6.

8. Composé selon la revendication 1, où R₄, R₅ et R₆ représentent chacun indépendamment un atome d'hydrogène ou un groupe (C₁-C₄) alkyle.

9. Composé selon la revendication 1, où A est un noyau de formule (a).

10. Composé selon la revendication 1, où R₇ et R₈ représentent chacun indépendamment un atome d'hydrogène ou un groupe (C₁-C₄) alkyle.

11. Composé selon la revendication 1, où A est une 1,4-benzodiazine de formule (i).

12. Composé selon les revendications de 1 à 11, sous la forme de ses dérivés N-oxyde.

13. Composé selon la revendication 1, choisi parmi
5-[2-(4-(3-trifluorométhyl-phényl)-1,2,3,6-tétrahydro-pyrid-1-yl)éthyl]-1H-indole,
6-[2-(4-(3-trifluorométhyl-phényl)-1,3,6-tétrahydro-pyrid-1-yl)éthyl]-1H-indole,
6-[2-(4-(3-trifluorométhyl-phényl)-1,2,3,6-tétrahydro-pyrid-1-yl)éthyl]-1H-benzimidazole,
7-[2-(4-(3-trifluorométhyl-phényl)-1,2,3,6-tétrahydro-pyrid-1-yl)éthyl]-N-méthyl-1,2,3,4-tétrahydro-isoquinoléine,
7-[2-(4-(3-trifluorométhyl-phényl)-1,2,3,6-tétrahydro-pyrid-1-yl)éthyl]-1,2,3,4-tétrahydro-isoquinoléine,
6-[2-(4-(3-trifluorométhyl-phényl)-1,2,3,6-tétrahydro-pyrid-1-yl)éthyl]-4-méthoxy-quinazoline,
6-[2-[4-(3-trifluoro-méthyl-phényl)-1,2,3,6-tétrahydro-pyrid-1-yl]-éthyl]-quinoxaline,
6-[2-[4-(3-trifluoro-méthyl-phényl)-1,2,3,6-tétrahydro-pyrid-1-yl]-éthyl]-2,3-diméthyl-quinoxaline,
6-[2-[4-(3-trifluoro-méthyl-phényl)-1,2,3,6-tétrahydro-pyrid-1-yl]-éthyl]-2,3-diéthyl-quinoxaline,
6-[2-[4-(6-trifluory-méthyl-pyrid-2-yl)-1,2,3,6-tétrahydro-pyrid-1yl]-éthyl]-quinoxaline,
6-[2-[4-(3-trifluoro-méthyl-phényl)-1,2,3,6-tétrahydro-pyrid-1-yl]-éthyl]-phtalazine,
leurs dérivés N-oxyde, leurs sels et solvates.

14. Procédé pour la préparation des composés de formule (I) selon la revendication 1, qui comprend la réaction d'un composé de formule (II) 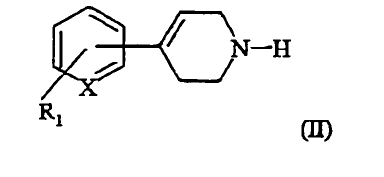 dans laquelle X et R₁ sont tels que définis ci-dessus, avec un composé de formule (III): 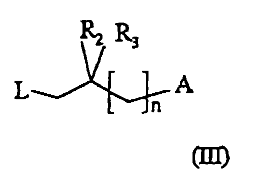 dans laquelle R₂, R₃, n et A sont tels que définis précédemment et L est un groupe partant, isolement du composé de formule (I) et transformation éventuelle en un de ses sels ou solvates ou dans ses dérivés N-oxydes.

15. Composition pharmaceutique contenant en tant que principe actif un composé de formule (I) selon les revendications de 1 à 13 ou un de ses sels ou solvates pharmaceutiquement acceptables.

16. Composition selon la revendication 15, **caractérisée en ce qu'**elle contient de 0,001 à 100 mg de principe actif.

17. Utilisation d'un composé de formule (I) 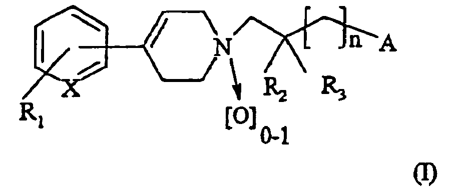 dans laquelle
X représente N ou CH ;
R₁ représente un atome d'hydrogène ou d'halogène ou un groupe CF₃;
R₂ et R₃ représentent indépendamment un atome d'hydrogène ou un groupe méthyle ;
n est 0 ou 1 ;
A représente un hétérocycle azoté de formule (a)-(1) : 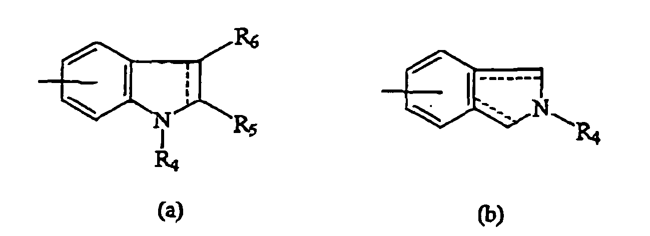 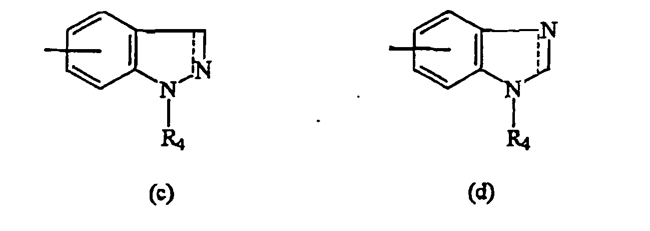 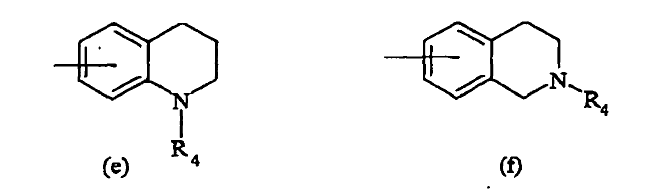 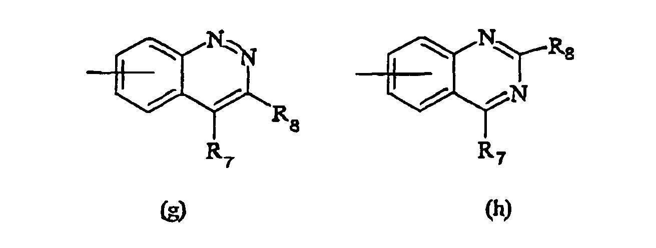 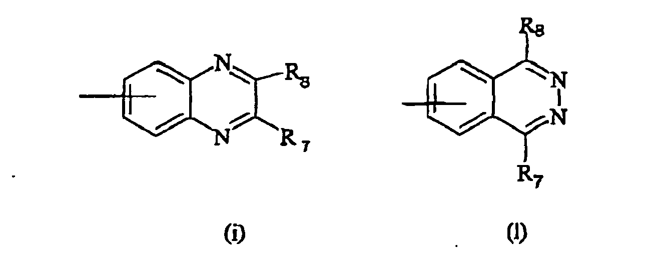
où R₄, R₅ et R₆ représentent chacun indépendamment un atome d'hydrogène ou un groupe (C₁-C₄) alkyle;
les lignes pointillées représentent des liaisons simples ou doubles;
R₇ et R₈ représentent chacun indépendamment un atome d'hydrogène ou d'halogène, un groupe (C₁-C₄) alkyle ou (C₁-C₄) alcoxy;
ou de ses N-oxydes ou d'un de ses sels ou solvates pharmaceutiquement acceptables, pour la préparation de médicaments destinés au traitement des maladies liées à des troubles immunitaires et inflammatoires.

18. Médicament contenant en tant que principe actif un composé de formule (I) selon les revendications de 1 à 13 ou un de ses sels ou solvates pharmaceutiquement acceptables.

## Claims

1. Compound of formula (I): 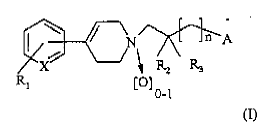 in which
X represents N or CH;
R₁ represents a hydrogen or halogen atom or a CF₃ group;
R₂ and R₃ independently represent a hydrogen atom or a methyl group;
n is 0 or 1;
A represents a nitrogenous heterocycle of formula (a)-(1): 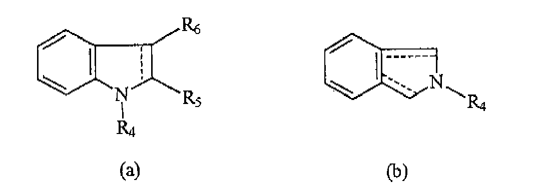 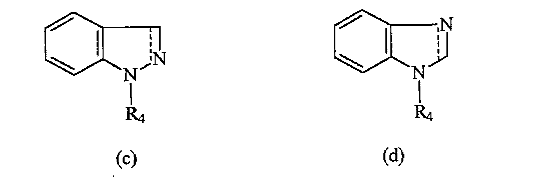 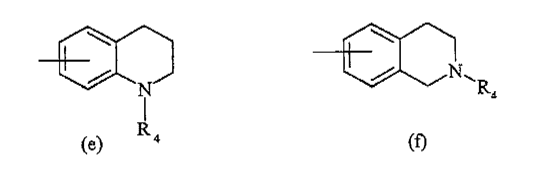 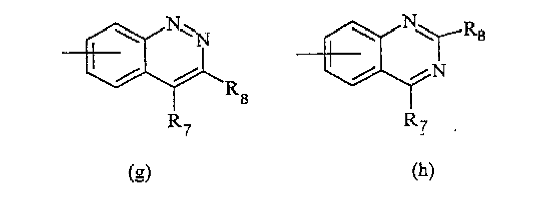 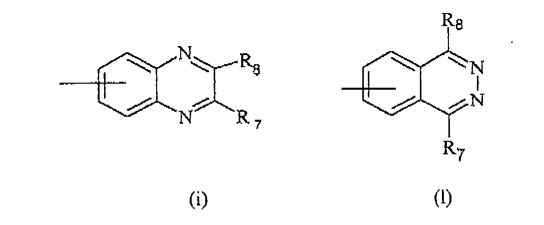
where R₄, R₅ and R₆ each independently represent a hydrogen atom or a (C₁-C₄)alkyl group;
the dotted lines represent single or double bonds;
R₇ and Rₐ each independently represent a hydrogen or halogen atom or a (C₁-C₄)alkyl or (C₁-C₄)alkoxy group;
and its N-oxides and its salts or solvates,
provided that, when X is CH, then (a) is not an indol-4-yl residue.

2. Compound according to Claim 1, where n is zero.

3. Compound according to Claim 1, where R₂ and R₃ are each a hydrogen.

4. Compound according to Claim 1, where R₁ is a CF₃ group.

5. Compound according to Claim 1, where X is CH and R₁ is in the 3-position of the benzene.

6. Compound according to Claim 1, where X is CH and R₁ is in the 2-position of the benzene.

7. Compound according to Claim 1, where X is N and the pyridine is substituted in the 2,6-positions.

8. Compound according to Claim 1, where R₄, R₅ and R₆ each independently represent a hydrogen atom or a (C₁-C₄)alkyl group.

9. Compound according to Claim 1, where A is a ring system of formula (a).

10. Compound according to Claim 1, where R₇ and R₈ each independently represent a hydrogen atom or a (C₁-Cy)alkyl group.

11. Compound according to Claim 1, where A is a 1,4-benzodiazine of formula (i).

12. Compound according to Claims 1 to 11, in the form of its N-oxide derivatives.

13. Compound according to Claim 1, chosen from
5-[2-(4-(3-(Trifluoromethyl)phenyl)-1,2,3,6-tetrahydra-pyrid-1-yl)ethyl]-1H-indole,
6-[2-(4-(3-(Trifluoromethyl)phenyl)-1,2,3,6-tetrahydro-pyrid-1-yl)ethyl]-1H-indole,
6-[2-(4-(3-(Trifluaromethyl)phenyl)-1,2,3,6-tetrahydro-pyrid-1-yl)ethyl]-1H-benzimidazole,
7-[2-(4-(3-(Trifluoromethyl)phenyl)-1,2,3,6-tetrahydro-pyrid-1-yl)ethyl]-N-methyl-1,2,3,4-tetrahydroiso-quinoline,
7-[2-(4-(3- (Trifluoromethyl)phenyl) -1,2,3,5-tetrahydro-pyrid-1-yl)ethyl]-1,2,3,4-tetrahydroisoquinoline,
6-[2-(4-(3-(Trifluoromethyl)phenyl)-1,2,3,6-tetrahydra-pyrid-1-yl)ethyl]-4-methoxyquinazoline,
6-[2-[4-(3-(Trifluoromethyl)phenyl)-1,2,3,6-tetrahydropyrid-1-yl]ethyl]quinoxaline,
6-[2-[4-(3-(Trifluoromethyl)phenyl)-1,2,3,6-tetrahydropyrid-1-yl]ethyl]-2,3-dimethylquinoxaline,
6-[2-[4-(3-(Trifluoromethyl)phenyl)-1,2,3,6-tetrahydropyrid-1-yl]ethyl]-2,3-diethylquinoxaline,
6-[2-[4-(6-(Trifluoromethyl)pyrid-2-yl)-1,2,3,6-tetra-hydropyrid-1-yl]ethyl]quinoxaline,
6-[2-[4-(3-(Trifluoromethyl)phenyl)-1,2,3,6-tetrahydro-pyrid-1-yl]ethyl]phthalazine,
their N-oxide derivatives and their salts and solvates.

14. Process for the preparation of the compounds of formula (I) according to Claim 1, which comprises the reaction of a compound of formula (II) 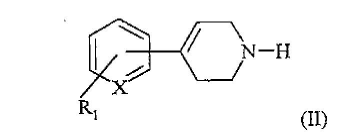 in which X and R₁ are as defined above, with a compound of formula (III): 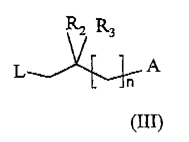 in which R₂, R₃, n and A are as defined above and L is a leaving group, isolation of the compound of formula (I) and optional conversion to one of its salts or solvates or to its N-oxide derivatives.

15. Pharmaceutical composition, comprising, as active principle, a compound of formula (I) according to Claims 1 to 13 or one of its pharmaceutically acceptable salts or solvates.

16. Composition according to Claim 15, which comprises from 0.001 to 100 mg of active principle.

17. Use of a compound of formula (I) 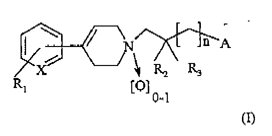 in which
X represents N or CH;
R₁ represents a hydrogen or halogen atom or a CF₃ group;
R₂ and R₃ independently represent a hydrogen atom or a methyl group;
n is 0 or 1;
A represents a nitrogenous heterocycle of formula (a)-(1): 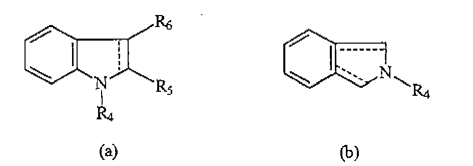 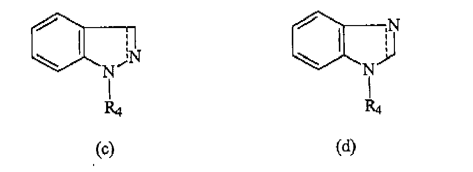 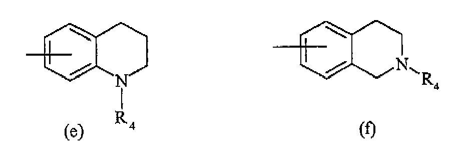 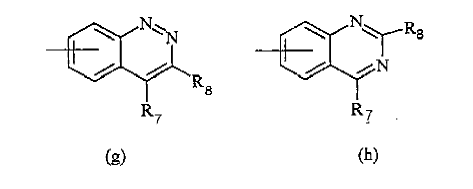 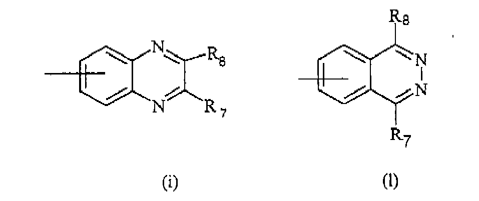
where R₄, R₅ and R₆ each independently represent a hydrogen atom or a (C₁-C₄)alkyl group;
the dotted lines represent single or double bonds;
R₇ and R₈ each independently represent a hydrogen or halogen atom or a (C₁-C₄)alkyl or (C₁-C₄)alkoxy group;
or of its N-oxides or of one of its pharmaceutically acceptable salts or solvates in the preparation of medicaments intended for the treatment of diseases related to immune and inflammatory disorders.

18. Medicament, comprising, as active principle, a compound of formula (I) according to Claims 1 to 13 or one of its pharmaceutically acceptable salts or solvates.

## Patentansprüche

1. Verbindung der Formel (I): 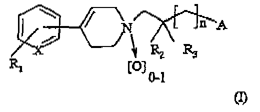 worin
X für N oder CH steht;
R₁ für ein Wasserstoff- oder Halogenatom oder eine CF₃-Gruppe steht;
R₂ und R₃ unabhängig voneinander für ein Wasserstoffatom oder eine Methylgruppe stehen;
n für 0 oder 1 steht;
A für einen stickstoffhaltigen Heterocyclus der Formel (a)-(1) steht: 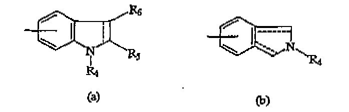 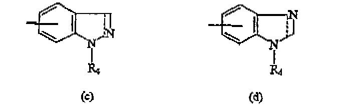 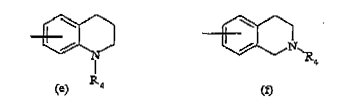 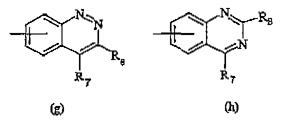 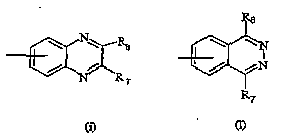
worin R₄, R₅ und R₆ jeweils unabhängig voneinander für ein Wasserstoffatom oder eine (C₁-C₄)-Alkylgruppe stehen;
die gestrichelten Linien für Einfach- oder Doppelbindungen stehen ;
R₇ und R₈ jeweils unabhängig voneinander für ein Wasserstoff- oder Halogenatom oder eine (C₁-C₄)-Alkyl- oder (C₁-C₄)-Alkoxygruppe stehen;
sowie N-Oxide davon und Salze oder Solvate davon,
mit der Maßgabe, daß (a) nicht für einen Indol-4-ylrest steht, wenn X für CH steht.

2. Verbindung nach Anspruch 1, worin n für null steht.

3. Verbindung nach Anspruch 1, worin R₂ und R₃ jeweils für Wasserstoff stehen.

4. Verbindung nach Anspruch 1, worin R₁ für eine CF₃-Gruppe steht.

5. Verbindung nach Anspruch 1, worin X für CH steht und R₁ in der 3-Position des Benzols steht.

6. Verbindung nach Anspruch 1, worin X für CH steht und R₁ in der 2-Position des Benzols steht.

7. Verbindung nach Anspruch 1, worin X für N steht und das Pyridin in den Positionen 2 und 6 substituiert ist.

8. Verbindung nach Anspruch 1, worin R₄, R₅ und R₆ jeweils unabhängig voneinander für ein Wasserstoffatom oder eine (C₁-C₄)-Alkylgruppe stehen.

9. Verbindung nach Anspruch 1, worin A für einen Ring der Formel (a) steht.

10. Verbindung nach Anspruch 1, worin R₇ und R₈ jeweils unabhängig voneinander für ein Wasserstoffatom oder eine (C₁-C₄)-Alkylgruppe stehen.

11. Verbindung nach Anspruch 1, worin A für ein 1,4-Benzodiazin der Formel (i) steht.

12. Verbindung nach einem der Ansprüche 1 bis 11 in Form ihrer N-Oxid-Derivate.

13. Verbindung nach Anspruch 1, ausgewählt unter
5-[2-(4-(3-Trifluormethylphenyl)-1,2,3,6-tetrahydropyrid-1-yl)ethyl]-1H-indol,
6-[2-(4-(3-Trifluormethylphenyl)-1,2,3,6-tetrahydropyrid-1-yl)ethyl]-1H-indol,
6-[2-(4-(3-Trifluormethylphenyl)-1,2,3,6-tetrahydropyrid-1-yl)ethyl]-1H-benzimidazol,
7-[2-(4-(3-Trifluormethylphenyl)-1,2,3,6-tetrahydropyrid-1-yl)ethyl]-N-methyl-1,2,3,4-tetrahydroisochinolin,
7-[2-(4-(3-Trifluormethylphenyl)-1,2,3,6-tetrahydropyrid-1-yl)ethyl]-1,2,3,4-tetrahydroisochinolin,
6-[2-(4-(3-Trifluormethylphenyl)-1,2,3,6-tetrahydropyrid-1-yl)ethyl]-4-methoxychinazolin,
6-[2-(4-(3-Trifluormethylphenyl)-1,2,3,6-tetrahydropyrid-1-yl)ethyl]chinoxalin,
6-[2-(4-(3-Trifluormethylphenyl)-1,2,3,6-tetrahydropyrid-1-yl)ethyl]-2,3-dimethylchinoxalin,
5-[2-(4-(3-Trifluormethylphenyl)-1,2,3,6-tetrahydropyrid-1-yl)ethyl]-2,3-diethylchinoxalin,
6-[2-(4-(6-Trifluormethylpyrid-2-yl)-1,2,3,6-tetrahydropyrid-1-yl)ethyl]chinoxalin,
6-[2-(4.-(3-Trifluormethylphenyl)-1,2,3,6-tetrahydropyrid-1-yl)ethyl]phthalazin,
N-Oxid-Derivaten und Salzen oder Solvaten davon.

14. verfahren zur Herstellung von Verbindungen der Formel (I) nach Anspruch 1, bei dem man eine Verbindung der Formel (II) 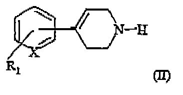 worin X und R₁ die oben angegebenen Bedeutungen besitzen, mit einer Verbindung der Formel (III): 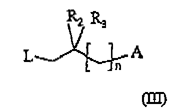 worin R₂, R₃, n und A die oben angegebenen Bedeutungen besitzen und L für eine Abgangsgruppe steht, die Verbindung der Formel (I) isoliert und gegebenenfalls in ein Salz oder Solvat davon oder in ein N-Oxid-Derivat davon umwandelt.

15. Pharmazeutische Zusammensetzung, enthaltend als Wirkstoff eine Verbindung der Formel (I) nach einem der Ansprüche 1 bis 13 oder ein pharmazeutisch unbedenkliches Salz oder Solvat davon.

16. Zusammensetzung nach Anspruch 15, **dadurch gekennzeichnet, daß** sie 0,001 bis 100 mg Wirkstoff enthält.

17. Verwendung einer Verbindung der Formel (I) 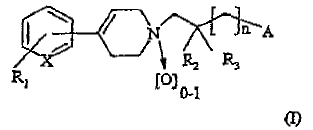 worin
X für N oder CH steht;
R₁ für ein Wasserstoff- oder Halogenatom oder eine CF₃-Gruppe steht;
R₂ und R₃ unabhängig voneinander für ein Wasserstoffatom oder eine Methylgruppe stehen;
n für 0 oder 1 steht;
A für einen stickstoffhaltigen Heterocyclus der Formel (a)-(1) steht: 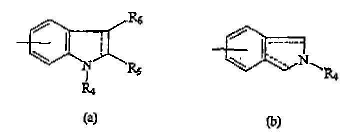 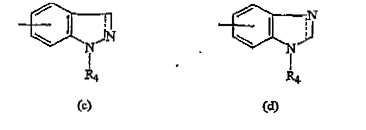 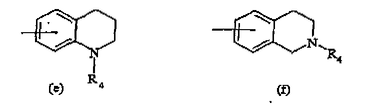 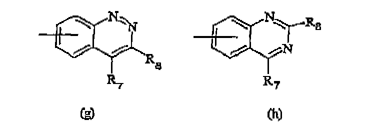 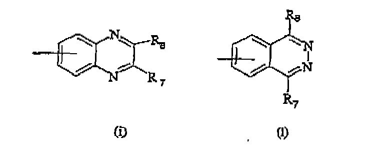
worin R₄, R₅ und R₆ jeweils unabhängig voneinander für ein Wasserstoffatom oder eine (C₁-C₄)-Alkylgruppe stehen;
die gestrichelten Linien für Einfach- oder Doppelbindungen stehen;
R₇ und R₈ jeweils unabhängig voneinander für ein Wasserstoff- oder Halogenatom oder eine (C₁-C₄)-Alkyl- oder (C₁-C₄)-Alkoxygruppe stehen;
oder N-Oxiden davon oder einem pharmazeutisch unbedenklichen Salz oder Solvat davon zur Herstellung von Arzneimitteln zur Behandlung von Erkrankungen, die mit Immunstörungen und inflammatorischen Störungen verbunden sind.

18. Arzneimittel, enthaltend als Wirkstoff eine Verbindung der Formel (I) nach einem der Ansprüche 1 bis 13 oder ein pharmazeutisch unbedenkliches Salz oder Solvat davon.
